# EUROPÄISCHE PATENTSCHRIFT

(11) **EP 4 474 644 B1**
(45) Veröffentlichungstag und Bekanntmachung des Hinweises auf die Patenterteilung: **18.06.2025**
(21) Anmeldenummer: 24179514.5
(22) Anmeldetag: 03.06.2024
(51) Int. Cl.: F04B 43/12, F04B 9/00, F04B 53/16, F04B 53/22, A61M 60/279

(54) **PERISTALTIKPUMPE**
PERISTALTIC PUMP
POMPE PÉRISTALTIQUE

(30) Priorität: 07.06.2023 DE 102023115075
(43) Veröffentlichungstag der Anmeldung: 11.12.2024
(73) Patentinhaber: B. Braun Avitum AG, 34212 Melsungen (DE)
(72) Erfinder: JAKOBI, Marco, 37318 Uder (DE)
(74) Vertreter: Winter, Brandl - Partnerschaft mbB

(56) Entgegenhaltungen:
- EP-B1- 1 749 549
- DE-A1- 102017 103 857
- DE-U1- 9 409 496
- US-A1- 2017 096 995
- US-B2- 9 200 628

## Beschreibung

### Technisches Gebiet

Die vorliegende Offenbarung betrifft eine Schlauchrollen- bzw. Peristaltikpumpe, d.h. eine Verdrängerpumpe, bei der das zu fördernde Medium durch äußere mechanische Verformung eines Schlauches durch diesen hindurchgedrückt wird, gemäß dem Oberbegriff des Patentanspruchs 1. Derartige Pumpen werden häufig zum Fördern von Fluid, insbesondere Blut, in einer Vorrichtung zur extrakorporalen Blutbehandlung, insbesondere in einer Dialysemaschine, verwendet. Das Fluid wird dabei mittels der Peristaltikpumpe von einer Niederdruckseite zu einer Hochdruckseite gefördert, wobei eine elastisch verformbare und zwischen der Niederdruckseite und der Hochdruckseite angeordnete Fluidleitung in Form eines Schlauchsegments, welches als Pumpensegment bezeichnet wird, zwischen einer Stützfläche eines Pumpenbetts und einem gegenüber dieser rotierenden Rotor mit zumindest zwei Quetschelementen verformt, insbesondere zusammengequetscht.

Gattungsgemäße Peristaltikpumpen sind in verschiedenen Ausführungen auf dem Markt. Bei einer beispielsweise von der Anmelderin des Dokuments EP 1 749 549 B1 hergestellten Pumpe, deren Aufbau aus der schematischen Schnittansicht gemäß Figur 8 entnehmbar ist, wird das Drehmoment von einer Antriebswelle 310 mit der Achse A mit Hilfe eines Freilaufes 320 und einer Rändelhülse 330 übertragen. Ein Rotor-Grundkörper 340 ist über eine kleine Gleitlagerstelle 350 am freien Ende der Antriebswelle auf dieser gelagert, wobei die Gleitlagerstelle 350 axial versetzt zur Rändelhülse 340 liegt, die sich nur bis zur Mitte des Rotor-Grundkörpers 340 erstreckt. Die Gleitlagerstelle 350 ist dabei in den Grundkörper 340 des Rotors integriert.

Um die Koaxialität zwischen Freilauf 310 und Lagerung 350 sicher zu stellen, müssen hohe Anforderungen an die Genauigkeit der Rändelhülse und des Grundkörpers gestellt werden. Weil ferner nur eine kleine einseitige Lagerung des Rotor-Grundkörpers 340 bereitgestellt ist, können nur bedingt radiale Kräfte und Kippmomente aufgenommen werden. Die Rändelhülse besitzt zwar über die gesamte Höhe eine Rändelung, aufgrund ihrer geringen axialen Erstreckung Höhe und der Montage im axial äußeren Bereich des Rotor-Grundkörpers 340 Grundkörpers wird das zu übertragende Drehmoment nur im unteren Bereich eingeleitet und bleibt daher relativ beschränkt.

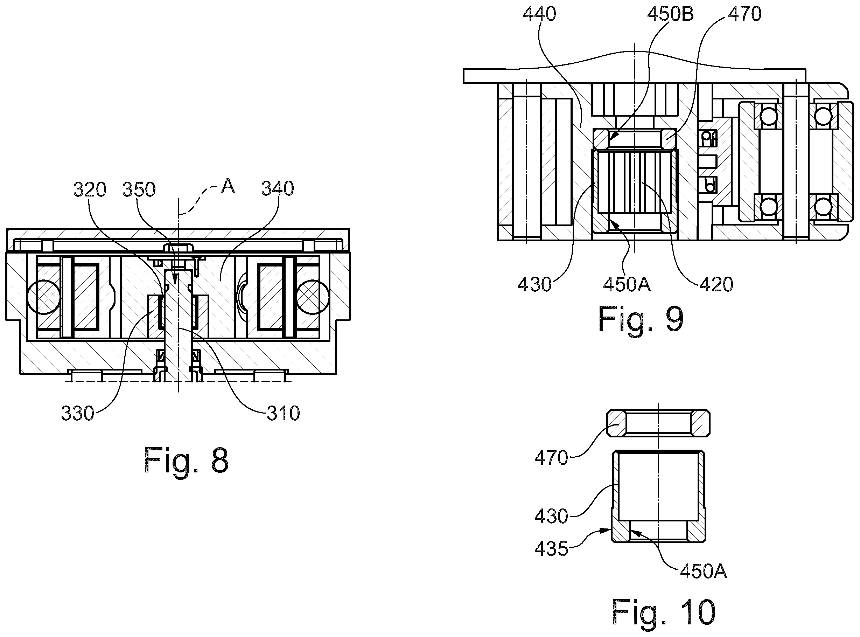

Es ist eine weitere, gattungsbildende Peristaltikpumpe mit den Merkmalen des Oberbegriffs des Anspruchs 1 bekannt, die wie in den Figuren 9 und 10 dargestellt aufgebaut ist. Die Figur 9 zeigt einen Teilschnitt eines Rotor-Grundkörpers mit eingepresstem Freilauf, während Figur 10 eine Schnittansicht einer dabei verwendeten Rändelhülse und eines Lagerrings zeigt. Bei dieser Peristaltikpumpe erfolgt die Demomentübertragung von der Antriebswelle auf den Rotor-Grundkörper 440 ebenfalls durch einen Freilauf 420, der in eine Rändelhülse 430 eingepresst ist. Die Rändelhülse 430 ist wiederum in eine zylindrische Ausnehmung des Rotor-Grundkörpers 440 eingepresst. Der Freilauf ist vorgesehen, um das manuelle Einfädeln und Ausfädeln des Pumpensegmentes sowie die manuelle Blutrückgabe zu ermöglichen, ohne dass eine Verriegelung geöffnet werden muss. Hülsenfreiläufe ohne Wälzlager sind Reibkupplungen und können nur Momente übertragen. Weil sie keine radialen Kräfte oder Kippmomente aufnehmen können, besitzt die Lagerung des Rotor-Grundkörpers 440 zwei Gleitlagerstellen, die mit den Bezugszeichen 450A und 450B bezeichnet sind. Die eine, randseitige Lagerstelle 450A ist in die Rändelhülse 430 unterhalb des Freilaufes integriert, wie in Figur 9 gezeigt. Radial außerhalb der Lagerstelle 450A, also dort, wo die Wandstärke der Rändelhülse 430 größer ist, ist die Rändelung 435 angebracht. Die zweite Lagerstelle 450B bildet ein gesonderter Lagerring 470, der gemäß Figur 8 oberhalb der Rändelhülse 430 in eine abgesetzte Bohrung des Rotor-Grundkörpers 440 Grundkörpers eingepresst ist. Dieser Aufbau erfordert hohe Fertigungsgenauigkeiten nicht nur bei den Teilen Rändelhülse 430 und Lagerring 470, sondern auch bei der Aufnahme im Rotor-Grundkörper 440, um die Koaxialität zwischen Freilauf 430 und Lagerung 450A,B sicherzustellen. Außerdem zeigt sich, dass auch bei diesem Aufbau das übertragbare Drehmoment beschränkt bleibt, weil es nur über die randseitig angeordnete Rändelung 435 eingeleitet werden kann.

Der Erfindung liegt die Aufgabe zugrunde, eine Peristaltikpumpe gemäß dem Oberbegriff des Anspruchs 1 zu schaffen, bei der unter Beibehaltung einer kippsicheren Lagerung des Rotor-Grundkörpers die Koaxialität zwischen Freilauf und Lagerung mit einem geringeren Fertigungsaufwand sichergestellt ist.

Diese Aufgabe wird mit den Merkmalen des Anspruchs 1 gelöst.

Bei der neu gestalteten Peristaltikpumpe werden zur Aufnahme radialer Kräfte und Kippmomente nach wie vor zwei Gleitlagerstellen vorgesehen. Diese sind allerdings so konzipiert, dass sie zu beiden Seiten des Freilaufs durch die Geometrie der fertig bearbeiteten Mitnehmerhülse festgelegt sind. Dadurch ergibt sich der besondere Vorteil, dass sich die hohen Anforderungen an die genaue Fertigung auf die Rändelhülse und einen gegebenenfalls einen darin aufgenommenen Lagerring beschränkt, um die Koaxialität zwischen Freilauf und Lagerung sicher zu stellen. Der Rotor-Grundkörper kann hingegen mit größeren Toleranzen gefertigt werden, wodurch sich der Fertigungsaufwand maßgeblich verringert. Darüber hinaus eröffnet diese Gestaltung die Möglichkeit, den Bauraum für die Gestaltung der Lagerstellen und der Drehmomentübertragung möglichst klein zu halten. Weil eine Lagerstelle von einem randseitigen, zylindrischen Innenoberflächenabschnitt der Mitnehmerhülse, und die andere Lagerstelle von einem in die Mitnehmerhülse mit Presspassung eingesetzten Lagerring gebildet sind, können die Lagerstellen in einem großen axialen Abstand angeordnet werden, wodurch es gelingt, die radialen Kräfte und Kippmomente auch mit Komponenten, wie z.B. einer Antriebswelle aufzunehmen, die einen verringertem Bauraum benötigen.

Vorteilhafte Ausgestaltungen sind Gegenstand der Unteransprüche.

Eine weitere Vereinfachung der Fertigung bei gleichzeitiger Sicherstellung einer hochpräzisen koaxialen Ausrichtung von Freilauf und Lagerung ergibt sich dann, wenn die Mitnehmerhülse eine durchgehende, d.h. gemeinsame Passungsfläche für den eingepressten Freilauf und den Lagerring hat.

Eine weitere vorteilhafte Ausgestaltung besteht darin, die Mitnehmerhülse außenseitig zumindest mit einem Verzahnungsabschnitt auszubilden, der zur Herstellung einer drehfesten Formschluss-Verbindung in eine zentrische, mit einer Entformschräge ausgebildeten Ausnehmung des Rotor-Grundkörpers eindrückbar ist. Auf diese Weise kann der Rotor-Grundkörper als Spritzgussteil ausgebildet werden, wobei die Ausnehmung im Rotor-Grundkörper keiner Nachbearbeitung mehr bedarf.

Wenn zwei axial beabstandete Verzahnungsabschnitte vorgesehen sind, die radial außerhalb der Lagerstellen liegen, kann das Drehmoment besonders gleichmäßig auf den Rotor-Grundkörper übertragen werden, wodurch sich das übertragbare Drehmoment bei kleiner Baugröße weiter anheben lässt.

Besonders vorteilhaft in Verbindung mit der Ausgestaltung des Rotor-Grundkörpers als Gussteil ist die Anordnung dann, wenn die Verzahnungsabschnitte unterschiedlich große Durchmesser haben, und die zentrische Ausnehmung des Rotor-Grundkörpers mehrere Teilbereiche mit unterschiedlichen Durchmessern und Entformschrägen aufweist. Dies erfüllt die Anforderungen an eine spritzgussgerechte Gestaltung. Außerdem kann hierdurch der Einpressvorgang der Rändelhülse vereinfacht, und der Winkel der erforderlichen Entformschräge optimiert werden.

Nachstehend werden anhand schematischer Zeichnungen Ausführungsbeispiele der Erfindung näher erläutert. Es zeigen:
Figur 1 eine perspektivische mittige Schnittansicht eines Rotors einer Peristaltikpumpe gemäß einer ersten Ausführungsform;
Figur 2 eine perspektivische Schnittansicht einer bei der Peristaltikpumpe gemäß Figur 1 verwendeten Rändelhülse mit eingepresstem Freilauf;
Figur 3 die Schnittansicht der Rändelhülse mit eingepresstem Freilauf gemäß Figur 2;
Figur 4 in vergrößertem Maßstab die Schnittansicht einer Aufnahme im Rotor-Grundkörper für die Rändelhülse gemäß Figur 2 und 3;
Figur 5 die Schnittansicht des Rotors der Peristaltikpumpe;
Figur 6 eine perspektivische, mittige Schnittansicht des Rotor-Grundkörpers der in den Figuren 1 bis 5 gezeigten Peristaltikpumpe;
Figur 7 die Schnittansicht einer modifizierten Ausgestaltung einer in den Rotor-Grundkörper gefügten Rändelhülse mit eingepresstem Freilauf;
Figur 8 eine schematische Schnittansicht einer Pumpe nach dem Stand der Technik;
Figur 9 einen schematischen Teilschnitt eines bekannten Rotor-Grundkörpers mit eingepresstem Freilauf; und
Figur 10 eine Schnittansicht einer bei der Pumpe gemäß Figur 9 verwendeten Rändelhülse und eines Lagerrings.

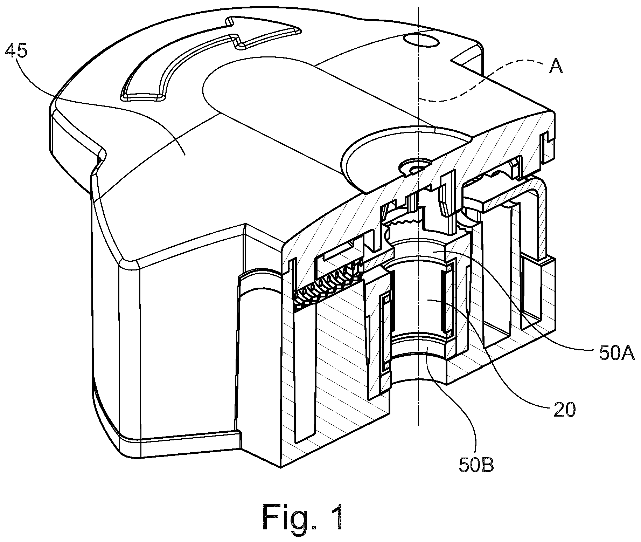

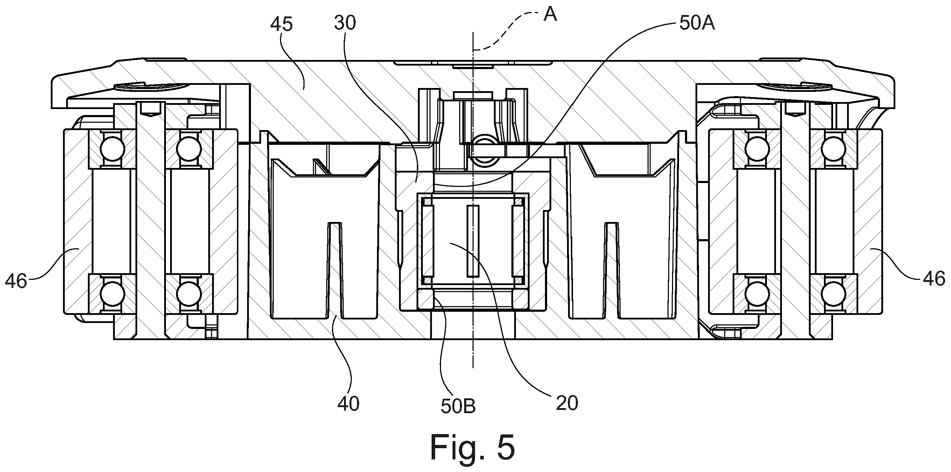

Figur 1 zeigt die mittige Schnittansicht eines Rotors 10 einer Peristaltikpumpe, die in einer Vorrichtung zur extrakorporalen Blutbehandlung, insbesondere in einer Dialysemaschine, eingesetzt werden kann. Die Peristaltikpumpe hat ein nicht näher gezeigtes Pumpengehäuse, in dem ein um eine Rotorachse A rotierbarer Rotor mit zumindest zwei in Umfangsrichtung zueinander versetzten Quetschelementen 46 (siehe Figur 5), die bei dem Ausführungsbeispiel gemäß Figur 1 bis 6 von Rollen gebildet sind, aufgenommen ist. Das Pumpengehäuse hat eine sich bogenförmig um die Rotorachse A erstreckende und radial von dem Rotor beabstandete Stützfläche, die zum Abstützen eines radial zwischen den Rotor und die Stützfläche einbringbaren Schlauchsegments eingerichtet ist.

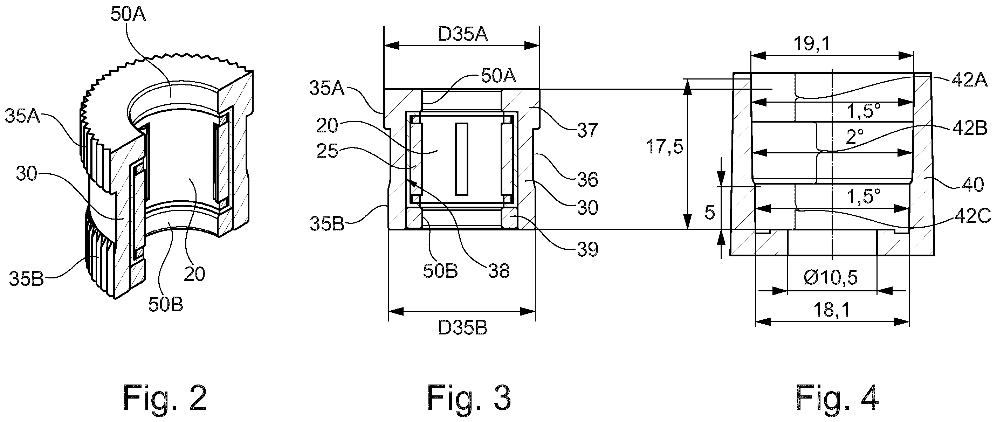

Im gezeigten Ausführungsbeispiel hat die Peristaltikpumpe eine nicht gezeigte Antriebswelle mit einer Drehachse A, mit der ein Rotor-Grundkörper 40, der eine Rotorabdeckung 45 trägt, antreibbar ist. Das Drehmoment der Antriebswelle wird über einen Hülsenfreilauf 20 (siehe Figur 2 und 3) auf den Rotor-Grundkörper 40 übertragen. Hierzu ist eine verzahnte Mitnehmerhülse, die im Folgenden als Rändelhülse 30 (siehe Figur 2) bezeichnet wird, vorgesehen, in die der Hülsenfreilauf 20 mit Freilaufrollkörpern 25 eingepresst ist. Der Freilauf ist vorgesehen, um das manuelle Einfädeln und Ausfädeln eines Pumpensegmentes sowie die manuelle Blutrückgabe zu ermöglichen, ohne dass eine Verriegelung geöffnet werden muss. Bei dem Hülsenfreilauf handelt es sich um einen Freilauf ohne Wälzlager und damit um eine Art Reibkupplung, mit der nur Momente übertragen werden können.

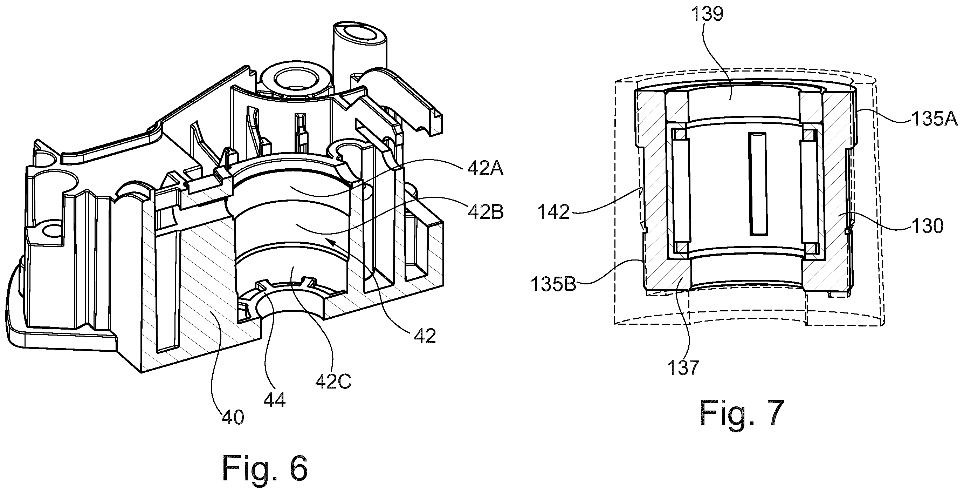

Die Rändelhülse 30 ist ihrerseits - wie den Figuren 1 und 5 entnehmbar - in den Grundkörper 40 des Rotors eingepresst, und zwar in eine zentrische Aufnahme 42 mit einer Anschlagschulter 44 (Figur 6). Zur verdrehsicheren Verbindung zum Rotor-Grundkörper 40 hat die Rändelhülse 30 zwei gerade Rändelungen 35A und 35B, die jeweils an den axialen Endabschnitten der Rändelhülse 30 ausgebildet sind. Im gezeigten Ausführungsbeispiel haben die Rändelungen 35A unterschiedliche Außendurchmesser D35A und D35B. Zwischen den Rändelungen 35A und 35B liegt eine glatte Eindrehung 36.

Zur Aufnahme radialer Kräfte und Kippmomente sind zu beiden Seiten des Hülsenfreilaufs 20 Gleitlagerstellen 50A und 50B vorgesehen. Die Gleitlagerstelle 50A ist von einer zylindrischen Ringfläche einer endseitigen Innenschulter 37 der Rändelhülse 30 gebildet. Die andere Gleitlagerstelle 50B wird von der Lauffläche eines in die Rändelhülse 30 eingepressten Lagerrings 39 gebildet. Auf diese Weise werden die Gleitlagerstellen 50A und 5B und deren Position zueinander zu beiden Seiten des Freilaufs 20 durch die Geometrie der fertig bearbeiteten Rändelhülse 30 festgelegt. Weil der Freilauf 20 ohnehin in eine Passungsfläche der Rändelhülse 30 eingepresst ist, kann die erforderliche Koaxialität zwischen Lagerstellen 50A, 50B und Freilauf 20 allein durch die Fertigung der Rändelhülse 30 sichergestellt werden. Mit anderen Worten legt allein die Fertigungsgenauigkeit der Rändelhülse 30 und des Lagerrings 39 die Koaxialität von Freilauf 20 und Lagerung des Rotor-Grundkörpers 40 fest, so dass der Rotor-Grundkörper 40 mit größeren Toleranzen gefertigt werden kann, wodurch sich der Fertigungsaufwand maßgeblich verringert. Darüber hinaus eröffnet dieses Konzept die Möglichkeit, den Bauraum für die Gestaltung der Lagerstellen und der Drehmomentübertragung möglichst klein zu halten.

Aus Figur 3 ist ersichtlich, dass der Lagerring 39 in eine zylindrische Füge-Passungsfläche 38 eingepresst ist, die gleichzeitig die Fügefläche für den Hülsenfreilauf 20 bildet. Dadurch kann die Herstellung weiter vereinfacht werden.

Weil der Rotor-Grundkörper 40 aufgrund der vorstehend beschriebenen Anordnung von Hülsenfreilauf 20 und Gleitlagerstellen 50A und 50B mit größeren Toleranzen hergestellt werden kann, kann er als Spritzgussteil, beispielsweise als metallisches oder als vorzugsweise glasfaserverstärktes Kunststoff-Spritzgussteil, ohne Nacharbeit ausgebildet werden. Die Ausgestaltung der Rändelungen 35A und 35B kommt dem wie folgt entgegen:
Weil die Rändelung 35A einen größeren Außendurchmesser als die Rändelung 35B hat, kann die Aufnahme 42 für die Rändelhülse 30 im Grundkörper - wie in Figur 4 dargestellt - in mehrere Teilbereiche 42A bis 42C mit unterschiedlichen Durchmessern und Entformschrägen aufgeteilt werden. Die Teilbereiche 42A und 42C sind leicht konisch ausgebildet, beispielsweise mit einem Konuswinkel von 1,5°. Diese Teilbereiche 42A und 42C sind hinsichtlich ihres Durchmessers und hinsichtlich ihrer axialen Erstreckung an die zugehörigen Rändelungen 35A und 35B derart angepasst, dass beim Eindrücken der Rändelhülse 30 mit ihren geraden Rändelungen 35A und 35B und einer damit einhergehenden Materialverdrängung eine gleichmäßig feste Verzahnung zwischen Rändelhülse und Rotor-Grundkörper 40 erfolgt, so dass das von der Rotorwelle eingeleitete Drehmoment möglichst gleichmäßig auf den Rotor-Grundkörper 40 im Wesentlichen über dessen gesamte Bauhöhe verteilt werden kann. Denn die formschlüssige Verbindung zwischen Rändelhülse 30 und Rotor-Grundkörper 40 liegt im Wesentlichen radial außerhalb der Lagerstellen 50A und 50B. Der zwischen den Teilbereichen 42A und 42C liegende Teilbereich 42B ist als Entformschräge mit einem etwas größeren Konuswinkel von 2° ausgebildet.

Bei dem vorstehend beschriebenen Ausführungsbeispiel ist die Anordnung so getroffen, dass die Rändelhülse 30 den Lagerring 39 auf der Seite trägt, auf der die Rändelung 35B mit kleinerem Durchmesser D35B ausgebildet ist. Die der Rotorabdeckung 45 zugewandte Lagerstelle 50A wird von der Rändelhülse 30 gebildet. Figur 7 zeigt eine modifizierte Ausgestaltung der Montageeinheit. In dieser Figur sind Komponenten, die Bauteilen des zuvor beschriebenen Ausführungsbeispiels entsprechen, mit ähnlichen Bezugszeichen versehen, denen eine "1" vorangestellt ist.

Hier ist die Innenschulter 137 der Rändelhülse 130 auf der Seite ausgebildet, auf der die Rändelung 135B mit kleinerem Durchmesser ausgebildet ist. Der Lagerring 139 ist auf der anderen Seite, d.h. radial innerhalb der Rändelung 135A mit größerem Durchmesser in die Füge-Passungsfläche der Rändelhülse 130 eingepresst. Die im Rotor-Grundkörper ausgebildete Aufnahme 142 entspricht der Aufnahme 42 des Ausführungsbeispiels nach den Figuren 1 bis 6.

Die Erfindung schafft somit eine Peristaltikpumpe, insbesondere zum Fördern von Fluid in einer Vorrichtung zur extrakorporalen Blutbehandlung, mit einem Pumpengehäuse, in dem ein um eine Rotorachse rotierbarer Rotor mit zumindest zwei in Umfangsrichtung zueinander versetzten Quetschelementen aufgenommen ist. Das Pumpengehäuse weist eine sich bogenförmig um die Rotorachse erstreckende und radial von dem Rotor beabstandete Stützfläche auf, die zum Abstützen eines radial zwischen den Rotor und die Stützfläche einbringbaren Schlauchsegments eingerichtet ist. Der Antrieb des Rotors erfolgt mittels einer Rotorwelle, deren Drehbewegung über einen in eine verzahnte Mitnehmerhülse eingepressten Freilauf auf einen Rotor-Grundkörper übertragbar ist, und zu beiden Seiten des Freilaufs eine Gleitlagerstelle für den Grundkörper vorgesehen ist. Um die Koaxialität zwischen Freilauf und kippsicherer Lagerung des Rotor-Grundkörpers mit einem geringen Fertigungsaufwand sicherzustellen, sind die Gleitlagerstellen zu beiden Seiten des Freilaufs durch die Geometrie der fertig bearbeiteten Mitnehmerhülse festgelegt.

### Bezugszeichenliste

- A: Achse
- 20: Hülsenfreilauf
- 25: Freilaufrollkörper
- 30: Rändelhülse
- 35A, B: Rändelungen
- 36: Eindrehung
- 37: Innenschulter
- 38: Füge-Passungsfläche
- 39: Lagerring
- 40: Rotor-Grundkörper
- 42: Aufnahme
- 42A, B, C: Teilbereiche von 42
- 44: Anschlagschulter
- 45: Rotorabdeckung
- 46: Quetschelemente
- 50A, B: Gleitlagerstellen

- 120: Freilauf
- 130: Rändelhülse
- 135A, B: Rändelungen
- 139: Lagerring

- 310: Antriebswelle
- 320: Freilauf
- 330: Rändelhülse
- 340: Rotor-Grundkörper
- 350: Lagerstelle

- 420: Hülsenfreilauf
- 430: Rändelhülse
- 435: Rändelung
- 450A, B: Gleitlagerstellen
- 470: Lagerring

## Patentansprüche

1. Peristaltikpumpe, insbesondere zum Fördern von Fluid in einer Vorrichtung zur extrakorporalen Blutbehandlung, mit einem Pumpengehäuse, in dem ein um eine Rotorachse (A) rotierbarer Rotor (40, 45) mit zumindest zwei in Umfangsrichtung zueinander versetzten Quetschelementen (46) aufgenommen ist und welches eine sich bogenförmig um die Rotorachse (A) erstreckende und radial von dem Rotor beabstandete Stützfläche aufweist, die zum Abstützen eines radial zwischen den Rotor und die Stützfläche einbringbaren Schlauchsegments eingerichtet ist, wobei der Antrieb des Rotors (40, 45) mittels einer Rotorwelle erfolgt, deren Drehbewegung über einen in eine verzahnte Mitnehmerhülse (30) eingepressten Freilauf (20) auf einen Rotor-Grundkörper (40) übertragbar ist, und zu beiden Seiten des Freilaufs (20) eine Gleitlagerstelle (50A, 50B) für den Rotor-Grundkörper (40) vorgesehen ist, **dadurch gekennzeichnet , dass** die Gleitlagerstellen (50A, 50B) zu beiden Seiten des Freilaufs (20) durch die Geometrie der fertig bearbeiteten Mitnehmerhülse (30) festgelegt sind, wobei eine Lagerstelle (50A; 150B) von einem randseitigen, zylindrischen Innenoberflächenabschnitt der Mitnehmerhülse (30; 130), und die andere Lagerstelle (50B; 150A) von einem in die Mitnehmerhülse (30; 130) mit Presspassung eingesetzten Lagerring (39; 139) gebildet ist.

2. Peristaltikpumpe nach Anspruch 1, **dadurch gekennzeichnet, dass** die Mitnehmerhülse (30) eine durchgehende Passungsfläche (38) für den eingepressten Freilauf (20) und den Lagerring (39) hat.

3. Peristaltikpumpe nach einem der Ansprüche 1 oder 2, **dadurch gekennzeichnet, dass** die Mitnehmerhülse (30) außenseitig zumindest einen Verzahnungsabschnitt (35A, 35B) hat, der in eine zentrische Ausnehmung (42) des Rotor-Grundkörpers (40) zur Herstellung einer drehfesten Formschluss-Verbindung eindrückbar ist.

4. Peristaltikpumpe nach Anspruch 3, **dadurch gekennzeichnet, dass** zwei axial beabstandete Verzahnungsabschnitte (35A, 35B; 135A, 135B) vorgesehen sind, die radial außerhalb der Lagerstellen (50A, 50B; 150A, 150B) liegen.

5. Peristaltikpumpe nach Anspruch 4, **dadurch gekennzeichnet, dass** die Verzahnungsabschnitte (35A, 35B; 135A, 135B) unterschiedlich große Durchmesser (D35A, D35B) haben, und die zentrische Ausnehmung (42) des Rotor-Grundkörpers (40) mehrere Teilbereiche (42A, 42B, 42C) mit unterschiedlichen Durchmessern und Entformschrägen aufweist.

6. Peristaltikpumpe nach Anspruch 5, **dadurch gekennzeichnet, dass** die Teilbereiche (42A, 42B, 42C) der zentrischen Ausnehmung (42) jeweils konisch mit gleichsinniger Konusöffnung ausgebildet sind.

7. Peristaltikpumpe nach Anspruch 6, **dadurch gekennzeichnet, dass** die Konuswinkel der die Verzahnungsabschnitte (35A, 35B; 135A, 135B) aufnehmenden Teilbereiche (42A, 42C) kleiner sind als der Konuswinkel des dazwischenliegenden Teilbereichs (42B).

8. Peristaltikpumpe nach einem der Ansprüche 1 bis 7, **dadurch gekennzeichnet, dass** der Rotor-Grundkörper (40) als Spritzgussteil, vorzugsweise aus glasfaserverstärktem Kunststoff oder Leichtmetall, ausgebildet ist.

## Claims

1. A peristaltic pump, specifically for conveying fluid in an apparatus for extracorporeal blood treatment, comprising
a pump housing in which a rotor (40, 45) rotatable about a rotor axis (A) and including at least two squeezing elements (46) offset against each other in the circumferential direction is accommodated and which includes a support surface extending in a curved shape around the rotor axis (A) and being radially spaced apart from the rotor, said support surface being arranged to support a tube segment to be radially inserted between the rotor and the support surface, wherein the rotor (40, 45) is driven by means of a rotor shaft, the rotary movement of which can be transmitted to a rotor base body (40) via a freewheel (20) press-fitted into a toothed driver sleeve (30), and a plain bearing position (50A, 50B) for the rotor base body (40) is provided on both sides of the freewheel (20), **characterized in that** the plain bearing positions (50A, 50B) are defined on both sides of the freewheel (20) by the geometry of the finished driver sleeve (30), wherein
one bearing position (50A; 150B) is formed by an edge-side cylindrical inner surface portion of the driver sleeve (30; 130) and the other bearing position (50B; 150A) is formed by a bearing ring (39; 139) inserted with press-fit into the driver sleeve (30; 130).

2. The peristaltic pump according to claim 1, **characterized in that** the driver sleeve (30) has a continuous fitting area (38) for the press-fitted freewheel (20) and the bearing ring (39).

3. The peristaltic pump according to one of the claims 1 or 2, **characterized in that** the driver sleeve (30) on the outside has at least one toothing section (35A, 35B) which can be pressed into a centric recess (42) of the rotor base body (40) to establish a rotationally fixed positive or form fit connection.

4. The peristaltic pump according to claim 3, **characterized in that** two axially spaced toothing sections (35A, 35B; 135A, 135B) are provided which are located radially outside the bearing positions (50A, 50B; 150A, 150B).

5. The peristaltic pump according to claim 4, **characterized in that** the toothing sections (35A, 35B; 135A, 135B) have differently sized diameters (D35A, D35B), and the centric recess (42) of the rotor base body (40) includes plural sections (42A, 42B, 42C) having different diameters and draft angles.

6. The peristaltic pump according to claim 5, **characterized in that** the sections (42A, 42B, 42C) of the centric recess (42) are each conical with a cone opening in the same direction.

7. The peristaltic pump according to claim 6, **characterized in that** the cone angles of the sections (42A, 42C) receiving the toothing sections (35A, 35B; 135A, 135B) are smaller than the cone angle of the intermediate section (42B).

8. The peristaltic pump according to one of the claims 1 to 7, **characterized in that** the rotor base body (40) is in the form of an injection-molded part, preferably made of glass-fiber reinforced plastic or light metal.

## Revendications

1. Pompe péristaltique, en particulier pour transporter du fluide dans un dispositif de traitement sanguin extracorporel, avec
un boîtier de pompe, dans lequel est logé un rotor (40, 45) pouvant tourner autour d'un axe de rotor (A) avec au moins deux éléments de pressage (46) décalés l'un par rapport à l'autre en direction circonférentielle et qui présente une surface d'appui s'étendant en forme d'arc autour de l'axe de rotor (A) et espacée radialement du rotor, surface d'appui qui est configurée pour soutenir un segment de tuyau pouvant être introduit radialement entre le rotor et la surface d'appui, dans laquelle l'entraînement du rotor (40, 45) s'effectue au moyen d'un arbre de rotor dont le mouvement de rotation peut être transmis à un corps de base de rotor (40) par l'intermédiaire d'une roue libre (20) enfoncée à la presse dans une douille d'entraîneur dentée (30), et un emplacement de palier lisse (50A, 50B) est prévu des deux côtés de la roue libre (20) pour le corps de base de rotor (40), **caractérisée en ce que** les emplacements de palier lisse (50A, 50B) des deux côtés de la roue libre (20) sont définis par la géométrie de la douille d'entraîneur (30) finie, dans laquelle un emplacement de palier (50A ; 150B) est formé par une section de surface intérieure cylindrique côté bord de la douille d'entraîneur (30 ; 130), et l'autre emplacement de palier (50B ; 150A) est formé par une bague de palier (39 ; 139) insérée dans la douille d'entraîneur (30 ; 130) avec un ajustement par serrage.

2. Pompe péristaltique selon la revendication 1, **caractérisée en ce que** la douille d'entraîneur (30) présente une surface d'ajustement continue (38) pour la roue libre (20) enfoncée à la presse et la bague de palier (39).

3. Pompe péristaltique selon l'une quelconque des revendications 1 ou 2, **caractérisée en ce que** la douille d'entraîneur (30) présente du côté extérieur au moins une section de denture (35A, 35B) qui peut être enfoncée dans un évidement central (42) du corps de base de rotor (40) pour réaliser une liaison par complémentarité de formes de manière non rotative.

4. Pompe péristaltique selon la revendication 3, **caractérisée en ce que** deux sections de denture (35A, 35B ; 135A, 135B) espacées axialement qui se situent radialement à l'extérieur des emplacements de palier (50A, 50B ; 150A, 150B) sont prévues.

5. Pompe péristaltique selon la revendication 4, **caractérisée en ce que** les sections de denture (35A, 35B ; 135A, 135B) présentent des diamètres (D35A, D35B) de différentes tailles, et l'évidement central (42) du corps de base de rotor (40) présente plusieurs zones partielles (42A, 42B, 42C) avec des diamètres et chanfreins de démoulage différents.

6. Pompe péristaltique selon la revendication 5, **caractérisée en ce que** les zones partielles (42A, 42B, 42C) de l'évidement central (42) sont conçues respectivement de forme conique avec une ouverture conique de même sens.

7. Pompe péristaltique selon la revendication 6, **caractérisée en ce que** les angles de cône des zones partielles (42A, 42C) logeant les sections de denture (35A, 35B ; 135A, 135B) sont plus petits que l'angle de cône de la zone partielle (42B) se situant entre les deux.

8. Pompe péristaltique selon l'une quelconque des revendications 1 à 7, **caractérisée en ce que** le corps de base de rotor (40) est conçu comme une pièce moulée par injection, de préférence en matière plastique renforcée par des fibres de verre ou en métal léger.
